# EUROPEAN PATENT APPLICATION

(11) **EP 4 335 869 A1**
(43) Date of publication of application: **13.03.2024**
(21) Application number: 23195781.2
(22) Date of filing: 06.09.2023
(51) Int. Cl.: C07K 16/06, C07K 1/22

(54) **RESIN AND CHROMATOGRAPHY COLUMN THAT PURIFIES ANTIBODIES WITH PROTEASE RESISTANT SMALL PEPTIDES**

(30) Priority: 07.09.2022 TR 202213896
(71) Applicant: Bio T Biyoteknoloji Cozumleri Ve Uretim Anonim, Istanbul (TR)
(72) Inventor: KOCAGOZ, Zuhtu Tanil, Istanbul (TR); CAN, Ozge, Istanbul (TR)
(74) Representative: Kaya, Erdem

(57) **Abstract**

Antibodies have been used in immunological tests for more than a century for the diagnosis of diseases. In recent years, they have also been used in the treatment of various diseases, primarily cancer and autoimmune diseases. These biological drugs have taken largest share of market of the pharmaceutical industry. Production of antibodies is largely accomplished by culturing the cells that produce these antibodies. One of the most important and most expensive steps of antibody production is the purification of the produced antibodies. For this purpose, a protein called "protein A" belonging to staphylococci, that binds antibodies, is used. The production of Protein A and the preparation of chromatography columns in which they are attached by binding to the resins to be used in purification, is a laborious and expensive process. While the antibody is purified in the columns, proteases from cell cultures break down protein A, causing it to lose its function in a short period of time. The present invention is the resins used in antibody purification and the chromatography columns using them, prepared with small peptides that will replace protein A, binding to antibodies as well as protein A, which are easy and inexpensive to produce and resistant to proteases. Protease-resistant antibody binding peptides are synthesized on these resins, which are then used directly in antibody purification.

## Description

### FIELD OF INVENTION

The present invention is a novel antibody purification tool. It is a resin where small peptide molecules resistant to proteases are bonded and which is easy to produce and which has low-cost and which is resistant and the chromatography column which contain these resins instead of protein A-linked resin used in purification of antibodies and which easily disrupts during use and which is difficult to produce and which has high cost, and the columns containing these resins. The present invention can be evaluated within the technical fields of biology and chemistry.

### DETAILED DESCRIPTION OF THE INVENTION AND THE DIFFERENCE THEREOF FROM

### METHODS KNOWN IN THE ART:

Antibodies are molecules produced by immune system cells and that bind to molecules (antigens) produced by infectious agents and cancer cells and that remove these and that protect the body. Since each antibody targets a specific molecule, it plays an important role in killing disease-causing cells without giving harm to other cells. In recent years, antibodies have been used in the treatment of many diseases, primarily cancers and autoimmune diseases. In this method, also called immunotherapy, a molecule found only in disease-causing cells is targeted. This molecule is purified, and cells that will form antibodies against it are obtained. The cells are then made immortal by fusing with myeloma (cancer) cells. The cell that produces the best antibody is selected from these cells, and then it is cultured in large volumes, and the antibody is obtained. Since these cells originate from a single cell, the antibodies produced by them are called monoclonal antibodies. In recent years, a significant share of the pharmaceutical market in the world has been taken by biological drugs in the form of monoclonal antibodies. In addition to being used for treatment, monoclonal antibodies are also used for the diagnosis of diseases, in immunological tests such as ELISA, immunochromatographic assays and immunofluorescent microscopy.

One of the most important steps in the production of monoclonal antibodies is the purification step of antibodies produced in cell cultures. For this purpose, a molecule called "protein A" found in staphylococci and that binds antibodies from their constant fragment (Fc -"Fraction constant"-) is used (CN113512099A Staphylococcus protein A as well as purification preparation method and application thereof). After protein A is produced by recombinant DNA technology, it is bound to a resin and is filled into a filtered column holding this resin. Cell culture fluids containing antibodies are passed through this column. Protein A binds to antibodies and keeps them in the column. Molecules other than antibodies are removed by washing the column with buffer solutions. In the final step, the antibodies are eluted from the column with solutions containing chemical molecules that are capable to disrupt the binding between antibodies and Protein A, and the antibodies are thus eluted from the column and collected. Thus, the most important step of antibody purification is completed.

The most effective method of antibody purification is affinity chromatography with protein A. However, protein A has significant disadvantages. Production of protein A with recombinant DNA technology, its purification and binding to resins is a task that requires significant effort and expense in itself (CN102329379A Recombined protein A, coding gene therein and purpose thereof). Therefore, protein A columns constitute one of the most important expense items in the production of monoclonal antibodies. The second important problem is that during antibody purification of protein A, proteases from the cells in which these antibodies are produced break down protein A and render it dysfunctional. For this reason, purification resins prepared with protein A and chromatography columns containing them become unusable in a short period of time.

Other chromatography methods have also been developed to purify antibodies without using protein A. For example, ion exchange resins that purify molecules based on electrical charge have been used for this purpose (US2016115195A1 Purification Process for Monoclonal Antibodies, WO2016162950A1 Separation and Purification Method for IgM Antibody). However, it is difficult to separate other molecules with similar charge from antibodies by this method. Antibody purification based on hydrophobic groups cannot perform a specific purification for antibodies by binding other proteins rich in hydrophobic groups (USRE41595E Antibody purification). Another method that is used is the separation of antibodies by means of thiol-linked resins. There are many disulfide bonds in the structure of antibodies. When these bonds meet with the thiol group, antibodies attach and hold to these groups (https://www.takarabio.com/products/protein-research/purification-products/ antibodypurification/thiophilic-resin). However, another problem is encountered here as well. Because many other proteins or molecules that can make disulfide bonds can also be attached to the resin carrying thiol groups. For this reason, resins carrying thiol groups cannot separate antibodies as well as protein A resins. Purification methods using a combination of several of the chromatography methods described above have also been described (US2011251374A1 ANTIBODY PURIFICATION METHOD). However, such methods make operations more complex, difficult and expensive.

The present invention includes a resin to which small synthetic protease resistant peptides which effectively bind antibodies are bonded, instead of protein A, a chromatography column in which this resin is placed, and an antibody purification method using these. For this purpose, firstly, peptides to be bound from the Fc part of the antibodies are synthesized by solid phase peptide synthesis, and afterwards, they are bound to a resin, and they are filled into a chromatography column with a filter which holds the resin thereunder. When the liquids or cell lysates containing the antibodies produced by the cells and secreted into the culture medium are passed through this column, the antibodies bind to the resin via peptides. The resin is thoroughly washed with washing solutions and then, solutions containing substances such as salt, urea, and glycine that will separate the antibodies from the peptides are passed through the column and the antibodies are eluted from the resin and come out of the column. In this way, antibodies are purified by separating them from all other molecules in the mixture. In a similar manner, in antibody purifications using protein A, protein A coated resins and chromatography columns filled with them become dysfunctional in a short period of time, since proteases from cell lysates degrade protein A. However, since the protease-resistant antibody binding peptides that we have developed, are not affected by proteases, antibody purification can be performed repeatedly with the resins to which they are attached. After the antibodies attached to the chromatography column are taken down from the column, the cell culture superfluid or cell lysate can be loaded again, and the antibodies therein can be separated by binding to the column, and this process can be repeated dozens of times. In our researches, we have determined that although a strong protease such as proteinase K is placed on the resin, the antibody binding peptides remained intact and continued their functions in the same way. Making peptides resistant to proteases can be accomplished by synthesizing the peptides from D-amino acids instead of L-amino acids. Additionally, we have determined that to make peptides completely resistant to proteases, either all amino acids in the peptide can be in the D form, or alternating as D and L form, sequentially.

Solid phase peptide synthesis is carried out by sequentially adding amino acids to the amino acid bound on a resin, using the methods described so far. After the synthesis of the peptide is completed, the peptide is separated from the resin and purified. The peptide molecule itself may be damaged during this separation (cutting) process (US5510460A Peptide process, US10683325B2 Methods and systems for solid phase peptide synthesis). When these peptides are to be used as a ligand in affinity chromatography, they must be re-linked to the resin. In order to eliminate these difficulties, in the present invention, we have developed the method of using resins on which peptide synthesis is made directly in antibody purification. In this method, after the protease-resistant peptide is synthesized on the resin, it is washed in order to remove the residual chemicals used in the synthesis, and the resin becomes ready for antibody purification. Thus, it is not needed to separate the peptide from one resin and attach it to another resin.

Although protease-resistant peptide ligands have been prepared previously (US10266566B2 Protease-resistant peptide ligands), antibody binding resins using protease resistant peptides have not been prepared and used in antibody purification to date.

### WORKING EXAMPLE ASSAY:

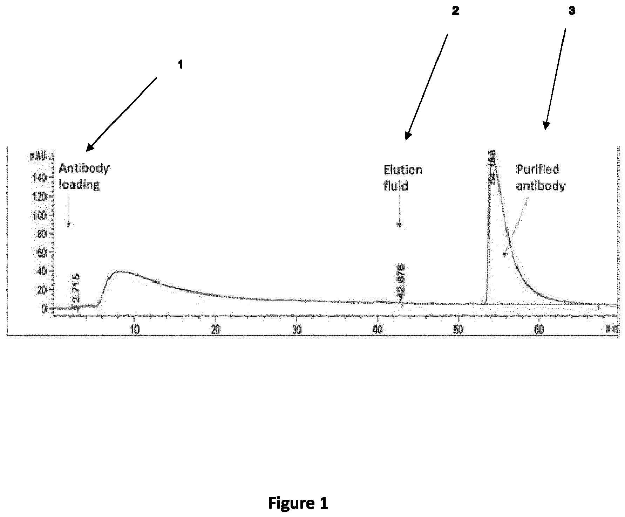

The 14 amino acid long peptide, which we have observed to bind to the antibody previously, was synthesized on the resin in a peptide synthesizer by solid phase peptide synthesis method. Most of the resin was separated for antibody purification, and the chemicals used in the synthesis were removed by washing with buffer solution. A small part was separated from the resin and purified by HPLC (high performance liquid chromatography). The peptide was incubated overnight at 37°C with proteinase K. When examined by HPLC again, it was observed that the peptide protected its original structure. Resin, prepared with protease resistant antibody binding peptide, was loaded into a column. After washing with the appropriate buffer solution, Anti-TNF-alpha monoclonal antibodies were loaded. Antibodies bound to the resin were removed from the column with a solution consisting of a mixture of glycine and NaCl (figure 1). After the column was washed, this process was repeated many times and it was seen that the antibodies could continue to be purified with the same efficiency. It was observed that passing the proteinase K solution through the column did not impair the antibody binding and purification efficiency of the column.

**Figure 1****.** Antibody purification with protease-resistant peptide-linked resin. Since the amount of antibody loaded at the 2.7^{th} minute (1) is slightly above the binding capacity of the column, it is seen that the antibodies that do not bind until approximately 20 minutes leave the column (2), and the antibodies leave the resin very quickly after the addition of elution liquid around the 43^{rd} minute (3).

## Claims

1. They are resins with peptide molecules resistant to proteases that bind antibodies in liquids containing antibodies and that enable separation of said antibodies from other molecules.

2. After the protease resistant peptides defined in claim 1 are synthesized on resins, the resins are used directly for antibody purification without separating the peptides from these resins and without binding to other resins.

3. The protease-resistant peptides defined in claim 1 are synthesized completely in D form or alternating one D, one L amino acids sequentially.

4. They are affinity chromatography columns used in antibody purification where the resins defined in claim 1 are filled.
